# EUROPÄISCHE PATENTSCHRIFT

(11) **EP 0 878 136 B1**
(45) Veröffentlichungstag und Bekanntmachung des Hinweises auf die Patenterteilung: **03.11.2004**
(21) Anmeldenummer: 98105947.0
(22) Anmeldetag: 01.04.1998
(51) Int. Cl.: A61L 2/26, A23L 3/04, A23L 3/00, B05B 1/20

(54) **Vorrichtung zur Flüssigkeitsbeaufschlagung von Gefässen**
Device for directing a fluid onto containers
Dispositif pour diriger un fluide sur des récipients

(30) Priorität: 23.04.1997 DE 29707271 U; 17.09.1997 DE 29716644 U
(43) Veröffentlichungstag der Anmeldung: 18.11.1998
(73) Patentinhaber: KRONES AG, 93068 Neutraubling (DE)
(72) Erfinder: Klenk, Klaus, 24939 Flensburg (DE)

(56) Entgegenhaltungen:
- WO-A-97/27931
- DE-A- 2 907 916
- DE-A- 19 734 485

## Beschreibung

Die Neuerung betrifft eine Vorrichtung zur Flüssigkeitsbeaufschlagung von Gefäßen mittels wenigstens einem Spritzrohr, welches über seine Längserstreckung verteilt eine Vielzahl von Austrittsöffnungen aufweist und durch einen Flüssigkeitszulauf gespeist wird.

Derartige Vorrichtungen werden in Tunnelpasteuren, Durchlaufkühl- oder Anwärmanlagen verwendet, um Lebensmittel oder Getränke enthaltende Dosen, Flaschen oder dgl. mit erwärmter oder gekühlter Flüssigkeit zu überrieseln. Bei Pasteuren soll durch eine genau definierte Energieübertragung eine vorgebbare Anzahl von sog. Pasteur-Einheiten (PE) erreicht werden. Eine zu hohe PE-Zahl kann den Lebensmittelgeschmack nachteilig beeinflussen, während eine zu geringe PE-Zahl keine ausreichende Haltbarkeit sicherstellt, d.h. es gilt, eine gleichmäßige Energieübertragung anzustreben. Der prinzipielle Aufbau derartiger Maschinen ist beispielsweise aus der DE-OS 2 142 124 (Fig. 1 und 2) oder US 4 704 958 (Fig. 1 und 3) bekannt.

Kühl- bzw. Anwärmanlagen für Flaschen oder Dosen sind dann erforderlich, wenn beispielsweise Getränke heiß oder kalt abgefüllt werden, weil sonst Probleme bei darauffolgenden Verpackungsvorgängen (Etikettieren, Folieneinhüllung) durch Schwitzwasserbildung o. dgl. auftreten können.

Üblicherweise werden die zu behandelnden Gefäße in den zuvor genannten Maschinen mehrspurig nebeneinander mittels einer Transporteinrichtung unter Spritzrohren vorbeigeführt, die mit ihrer Längserstreckung quer zur Transportrichtung ausgerichtet sind und entlang ihrer Unterseite eine Vielzahl von Spritzöffnungen oder Düsen aufweisen. Eine Ausführung eines entsprechenden Spritzrohres ist aus der deutschen Patentschrift 29 07 916 C2 bekannt. Das in diesem Dokument beschriebene Spritzrohr weist einen über seine gesamte Länge gleichbleibenden Querschnitt auf. Durch eine Zuleitung wird das Spritzrohr von einem Ende her in axialer Richtung mit Flüssigkeit gespeist. Nachteiligerweise ist die Flüssigkeitsausbringung der einzelnen vom Zuleitungsanschluß unterschiedlich weit entfernten Spritzöffnungen aufgrund der ungleichen Mengenverteilung und Druckverhältnisse im Rohrinnenraum nicht einheitlich.

Der Neuerung liegt demzufolge die Aufgabe zugrunde, ein Spritzrohr mit verbesserter Ausbringung anzugeben.

Gelöst wird diese Aufgabe durch den Gegenstand des Anspruchs 1, bei dem ein mit zunehmender Entfernung vom Flüssigkeitszulauf abnehmender Innenquerschnitt des Spritzrohres definiert wird. Dadurch wird eine gleichmäßige Flüssigkeitsabgabe sichergestellt, unabhängig vom jeweiligen Abstand der Austrittsöffnungen zum Zuleitungsanschluß. Das Maß der Innenquerschnittsabnahme bezogen auf die Entfernung vom Zuleitungsanschluß hängt vom Zwischenabstand der Austrittsöffnungen, d.h. der Anzahl von Öffnungen pro Längeneinheit, deren Ausflußvermögen und ggf. auch den Rohrreibungsverlusten ab und ist so bemessen, dass der im Spritzrohr vorhandene Gesamtdruck im Bereich jeder Austrittsöffnung annähernd gleich groß ist.

Wenn die Austrittsöffnungen in regelmäßigen Abständen entlang der Längserstreckung eines Spritzrohres verteilt angeordnet sind und einen übereinstimmenden Öffnungsquerschnitt aufweisen, ist ein mit zunehmendem Abstand vom Zuleitungsanschluß stetig abnehmender Spritzrohrinnenquerschnitt vorteilhaft. Ein derartiges Spritzrohr kann beispielsweise mit einem sich vom Zuleitungsanschluß aus keil- oder pyramidenförmig verjüngenden Innenraum versehen sein.

Erfolgt die Flüssigkeitszufuhr nicht über eines der Enden des Spritzrohres, sondern im dazwischenliegenden Bereich, so befindet sich der maximale Innenquerschnitt an der Zuleitungsanschlußstelle und nimmt von dort zu den Enden des Spritzrohres hin ab. Genau entgegengesetzt verhält sich der Innenquerschnittsverlauf eines Spritzrohres, das von beiden Enden gleichzeitig durch jeweils eine Zuleitung gespeist wird. Hier nimmt der Innenquerschnitt von den Rohrenden zur Mitte hin ab.

Nachfolgend wird eine bevorzugte Ausführung eines Spritzrohres anhand der Figuren erläutert: Es zeigt:
- Fig. 1: eine perspektivische Darstellung eines Spritzrohres,
- Fig. 2: einen Vertikalschnitt durch ein Spritzrohr und
- Fig. 3: eine Ansicht auf das dem Flüssigkeitszulauf gegenüberliegende Ende des Spritzrohres nach Fig. 1.

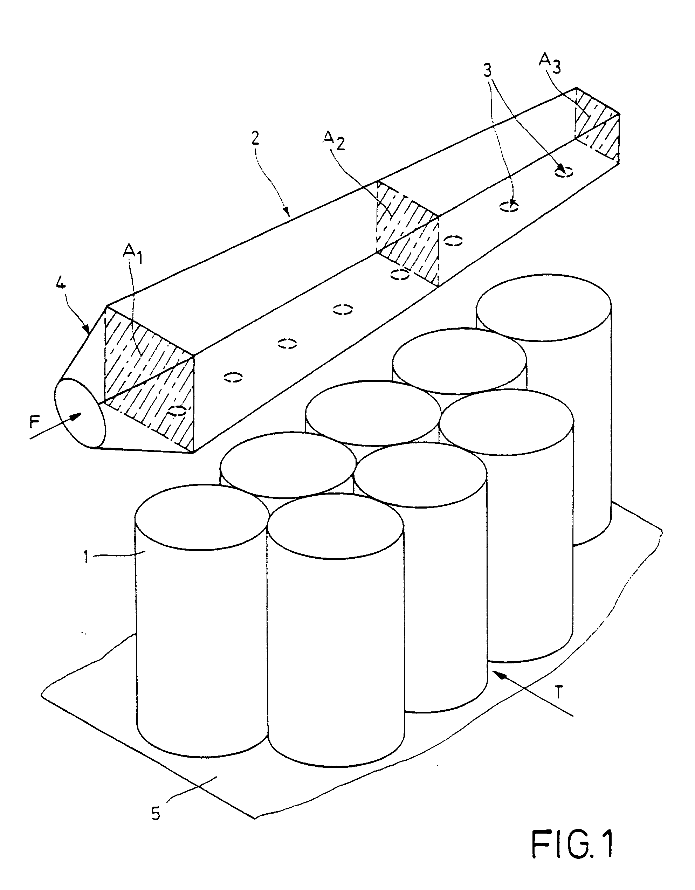

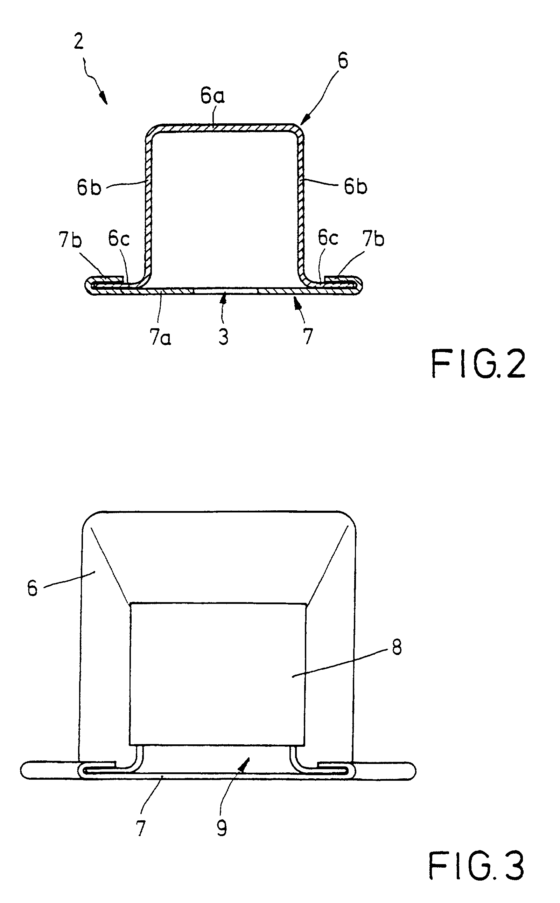

Das in Fig. 1 nur schematisch dargestellte Spritzrohr 2 ist mit seiner Längserstreckung quer zur Bewegungsrichtung T eines von einem Transporteur 5 mehrspurig geförderten Dosenstromes ausgerichtet. An der zu den Dosen 1 weisenden Unterseite des Spritzrohres 2 befinden sich in regelmäßigen Abständen mehrere gleich große Austrittsöffnungen 3. An einem Ende ist ein Anschlußstück 4 für den Flüssigkeitszulauf angeordnet, während das gegenüberliegende Rohrende bis auf eine kleine Spaltöffnung (Fig. 3) zum Ausschneiden von Schmutzpartikeln verschlossen ist. Die Flüssigkeit wird in Pfeilrichtung F eingespeist.

Der Spritzrohrinnenraum weist eine pyramidenstumpfförmige Kontur auf, wobei das die größte Innenquerschnittsfläche A1 aufweisende Rohrende dem Flüssigkeitszulauf 4 zugeordnet ist. Von dieser Anschlußstelle 4 aus strömt die Flüssigkeit in axialer Richtung in den sich fortlaufend verjüngenden Rohrinnenraum.

Wie aus der Fig. 1 weiter zu erkennen ist, nimmt die Innenquerschnittsfläche A1 des Spritzrohres 2 in Fließrichtung F gesehen mit zunehmender Entfernung vom anschlußseitigen Rohrende in Richtung zum gegenüberliegenden Ende stetig ab. Die Querschnittsabnahme (A1 bis A3) ist konstruktiv so getroffen, dass der Gesamtdruck (statischer und dynamischer Flüssigkeitsdruck) im Spritzrohr 2 im Bereich jeder Austrittsöffnung 3 annähernd gleich groß ist. Damit wird eine gleichmäßige Flüssigkeitsabgabe unabhängig vom Abstand jeder einzelnen Austrittsöffnung 3 zur Flüssigkeitszuleitungsstelle 4 erreicht. Insbesondere bei einer Verwendung in Tunnelpasteuren ist diese Eigenschaft solcher Spritzrohre von großer Bedeutung, weil hierdurch vorteilhafterweise eine konstante Wärmeübertragung sichergestellt wird, ganz gleich ob nun die Lebensmittel oder Getränke enthaltenden Flaschen, Dosen oder dgl. näher im Bereich des anschlußnahen oder -fernen Endes des Spritzrohres 2 vorbeigeführt werden. Das neuerungsgemäße Spritzrohr kann auch in Flaschenreinigungsmaschinen verwendet werden, wo die Beaufschlagung aller Flaschen mit der gleichen Reinigungsmittelmenge ebenfalls von Vorteil ist.

Ein Spritzrohr mit einem sich in axialer Richtung verjüngenden Innenraum kann durch Biegen eines entsprechend zugeschnittenen Blechteils hergestellt werden. Eine besonders einfach herstellbare Bauform ist in der Fig. 2 dargestellt. Wie zu erkennen ist, besteht das Spritzrohr 2 aus zwei zusammengesetzten Einzelteilen 6 und 7, nämlich einem ersten Blechzuschnitt 6, der durch vier nicht parallel verlaufende Biegekanten einen im wesentlichen U-förmigen, unten offenen Querschnitt aufweist, und einem zweiten, das Boden- bzw. Unterteil des Spritzrohres bildenden Blechzuschnitt 7. Der erste, das Oberteil des Spritzrohres bildende Blechzuschnitt 6 besitzt zwei von einer Oberseite 6a vertikal nach unten abgebogene Seitenflächen 6b, die an ihrem unteren Ende nahtlos in rechtwinklig nach außen abstehende Flanschflächen 6c übergehen. In den zweiten, trapezförmigen Blechzuschnitt 7 sind die Auslauföffnungen 3 eingestanzt. Im gestreckten Zustand besitzt dieser Blechzuschnitt 7 eine größere Breite als das U-förmige Blechteil 6 im Bereich seiner Flanschflächen 6c, so dass der zweite Blechzuschnitt 7 an seinen Längsseiten durch Umbördeln mit den Flanschflächen 6c formschlüssig entsprechend der Darstellung in Fig. 2 verbunden werden kann.

Die dem Flüssigkeitsanschluß 4 (Fig. 1) gegenüberliegende Stirnseite des Spritzrohres 2 ist mit einer angeschweißten Platte 8 versehen (Fig. 3), die den Rohrquerschnitt bis auf einen schmalen Spalt 9 abdeckt. Durch den Spalt 9 können Fremdkörper aus dem Rohrinnenraum ins Freie gespült werden. Aus der Fig. 3 ist ferner ersichtlich, dass nicht nur die vertikalen Seitenflächen des Oberteils 6 vom Flüssigkeitsanschluß aus in Richtung zur Platte 8 verjüngend zusammenlaufen, sondern auch die Oberseite relativ zu dem horizontal ausgerichteten Unterteil 7 abfällt, d.h. das Spritzrohr eine pyramidenstumpfähnliche Gehäusekontur besitzt.

## Patentansprüche

1. Vorrichtung zur Flüssigkeitsbeaufschlagung von Gefäßen (1) mit wenigstens einem Spritzrohr (2), welches über seine Längserstreckung verteilt eine Vielzahl von Austrittsöffnungen (3) aufweist und durch einen Flüssigkeitszulauf (4) gespeist wird, und einem Transporteur (5) zum Vorbeiführen der Gefäße an dem Spritzrohr, **dadurch gekennzeichnet, dass** der Innenraum des Spritzrohres (2) keil-, pyramiden- oder pyramidenstumpfförmig verlaufend ausgebildet ist, derart, dass der Innenquerschnitt (A1, A2, A3) des Spritzrohres (2) mit zunehmender Entfernung vom Flüssigkeitszulauf (4) abnimmt.

2. Vorrichtung nach einem der Ansprüche 1, **dadurch gekennzeichnet, dass** der Flüssigkeitszulauf (4) an einem Ende des Spritzrohres (2) erfolgt und das gegenüberliegende Ende (8) teilweise verschlossen ist.

3. Vorrichtung nach einem der Ansprüche 1, **dadurch gekennzeichnet, dass** beide Enden des Spritzrohres teilweise verschlossen sind und sich der Flüssigkeitszulauf im Bereich zwischen den Enden befindet.

4. Vorrichtung nach wenigstens einem der Ansprüche 1 bis 3, **dadurch gekennzeichnet, dass** das Spritzrohr (2) mit seiner Längserstreckung quer zur Transportrichtung (T) der Gefäße (1) ausgerichtet ist.

5. Vorrichtung nach wenigstens einem der vorhergehenden Ansprüche 1 bis 4, **dadurch gekennzeichnet, dass** mehrere Spritzrohre (2) parallel in einer Ebene angeordnet sind, insbesondere in einer zur Gefäßtransportebene (5) parallelen Ebene.

6. Vorrichtung nach wenigstens einem der vorhergehenden Ansprüche 1 bis 5, **dadurch gekennzeichnet, dass** dem Spritzrohr (2) eine erwärmte oder gekühlte Flüssigkeit zugeführt wird.

7. Vorrichtung nach wenigstens einem der vorhergehenden Ansprüche 1 bis 6, **dadurch gekennzeichnet, dass** das Spritzrohr (2) Bestandteil eines Pasteurs, einer Gefäßwaschmaschine, eines Gefäßkühl- oder -wärmapparats oder Rinsers zur Außenspritzung von Gefäßen (1) ist.

8. Vorrichtung nach wenigstens einem der vorhergehenden Ansprüche 1 bis 7, **dadurch gekennzeichnet, dass** das Spritzrohr (2) Bestandteil einer Vorrichtung zum Behandeln von Flaschen oder Dosen (1) ist.

9. Vorrichtung nach wenigstens einem der vorhergehenden Ansprüche 1 bis 8, **dadurch gekennzeichnet, dass** das Spritzrohr (2) ein sich in Längsrichtung verjüngendes Gehäuse aufweist, welches aus einem gebogenen Blechzuschnitt gebildet wird.

10. Vorrichtung nach wenigstens einem der vorhergehenden Ansprüche 1 bis 9, **dadurch gekennzeichnet, dass** das Spritzrohr (2) ein im wesentlichen aus zwei Einzelteilen (6, 7), insbesondere Blechzuschnitten, bestehendes Gehäuse aufweist, wobei die Einzelteile (6, 7) vorzugsweise durch Formschluss verbunden sind.

11. Vorrichtung nach Anspruch 10, **dadurch gekennzeichnet, dass** das Gehäuse des Spritzrohres (2) ein Oberteil (6) mit einem im wesentlichen U-förmigen, unten offenen Querschnitt mit seitlich nach außen ragenden Flanschflächen (6c) und ein im wesentlichen ebenes, plattenförmiges Unterteil (7) aufweist, dessen äußeren Längsabschnitte (7b) die Flanschflächen (6c) des Oberteils (6) formschlüssig umfassen.

12. Vorrichtung nach Anspruch 11, **dadurch gekennzeichnet, dass** sich die Austrittsöffnungen (3) im Unterteil (7) befinden.

## Claims

1. Device for applying liquid to containers (1) having at least one spray tube (2) which has a multiplicity of outlet openings (3) distributed along its length and is fed by a liquid supply (4), and having a conveyor (5) for moving the containers past the spray tube, **characterised in that** the interior of the spray tube (2) is constructed extending in the shape of a wedge or pyramid or truncated pyramid, such that the inner cross section (A1, A2, A3) of the spray tube (2) decreases at increasing distance from the liquid supply (4).

2. Device according to one of claims 1, **characterised in that** the liquid supply (4) is effected at one end of the spray tube (2) and the opposite end (8) is partly closed.

3. Device according to one of claims 1, **characterised in that** both ends of the spray tube are partly closed and the liquid supply is disposed in the region between the ends.

4. Device according to at least one of claims 1 to 3, **characterised in that** the spray tube (2) is arranged with its length extending transversely with respect to the conveying direction (T) of the containers (1).

5. Device according to at least one of the preceding claims 1 to 4, **characterised in that** a plurality of spray tubes (2) are arranged in parallel in a plane, especially in a plane that is parallel to the plane (5) in which the containers are conveyed.

6. Device according to at least one of the preceding claims 1 to 5, **characterised in that** the spray tube (2) is supplied with liquid that is heated or cooled.

7. Device according to at least one of the preceding claims 1 to 6, **characterised in that** the spray tube (2) forms part of a pasteuriser or of a machine for washing containers or of apparatus for cooling or heating containers or of a rinser for spraying containers (1) from the outside.

8. Device according to at least one of the preceding claims 1 to 7, **characterised in that** the spray tube (2) forms part of a device for the treatment of bottles or cans (1).

9. Device according to at least one of the preceding claims 1 to 8, **characterised in that** the spray tube (2) has a tapered housing narrowing in the longitudinal direction formed of a bent sheet metal blank.

10. Device according to at least one of the preceding claims 1 to 9, **characterised in that** the spray tube (2) has a housing consisting essentially of two components (6, 7), especially sheet metal blanks, the components (6, 7) being preferably connected by positive engagement.

11. Device according to claim 10, **characterised in that** the housing of the spray tube (2) has an upper part (6) with an essentially U-shaped cross section, open at the bottom, with flange surfaces (6c) that project laterally outwards, and has an essentially flat plate shaped lower part (7) whose outer longitudinal sections (7b) embrace the flange surfaces (6c) of the upper part (6) with positive engagement.

12. Device according to claim 11, **characterised in that** the outlet openings (3) are disposed in the lower part (7).

## Revendications

1. Dispositif pour projeter un liquide sur des récipients, comprenant au moins une lance de projection (2) qui présente une multitude d'ouvertures de sortie (3) réparties sur son étendue longitudinale, alimenté par une admission de liquide (4), et comprenant un dispositif de transport (5) pour faire passer les récipients devant la lance de projection,
**caractérisé en ce que**
l'intérieur de la lance de projection (2) présente la forme d'un coin, d'une pyramide ou d'une pyramide tronquée, de telle sorte que la section transversale intérieure (A1, A2, A3) de la lance de projection (2) diminue au fur et à mesure de l'éloignement de l'admission de liquide (4).

2. Dispositif selon la revendication 1,
**caractérisé en ce que**
l'admission de liquide (4) est réalisée à une extrémité de la lance de projection (2), l'extrémité opposée (8) étant partiellement fermée.

3. Dispositif selon la revendication 1,
**caractérisé en ce que**
les deux extrémités de la lance de projection sont partiellement fermées, l'admission de liquide se situant dans la zone entre les extrémités.

4. Dispositif selon au moins l'une des revendications 1 à 3,
**caractérisé en ce que**
la lance de projection (2) est orientée, par son étendue longitudinale, transversalement à la direction de transport (T) des récipients (1).

5. Dispositif selon au moins l'une des revendications 1 à 4,
**caractérisé en ce que**
plusieurs lances de projection (2) sont disposées en parallèle dans un plan, notamment dans un plan parallèle au plan de transport de récipients (5).

6. Dispositif selon au moins l'une des revendications 1 à 5,
**caractérisé en ce qu'**
un liquide chauffé ou refroidi est amené à la lance de projection (2).

7. Dispositif selon au moins l'une des revendications 1 à 6,
**caractérisé en ce que**
la lance de projection (2) fait partie d'un pasteurisateur, d'une machine à laver les récipients, d'un appareil de refroidissement ou de chauffage de récipients ou d'un rinceur pour la projection sur l'extérieur de récipients (1).

8. Dispositif selon au moins l'une des revendications 1 à 7,
**caractérisé en ce que**
la lance de projection (2) fait partie d'un dispositif de traitement de bouteilles ou de boîtes (1).

9. Dispositif selon au moins l'une des revendications 1 à 8,
**caractérisé en ce que**
la lance de projection (2) présente un boîtier s'amincissant dans la direction longitudinale, qui est réalisé à partir d'un flan en tôle courbe.

10. Dispositif selon au moins l'une des revendications 1 à 9,
**caractérisé en ce que**
la lance de projection (2) présente un boîtier composé pour l'essentiel de deux éléments distincts (6, 7), notamment de flans en tôle, les éléments distincts (6, 7) étant de préférence assemblés par complémentarité de formes.

11. Dispositif selon la revendication 10,
**caractérisé en ce que**
le boîtier de la lance de projection (2) présente une partie supérieure (6) d'une section transversale pour l'essentiel en forme d'un U ouvert vers le bas avec des surfaces de bridage (6c) saillant latéralement vers l'extérieur, et une partie inférieure (7) pour l'essentiel plane en forme de plaque, dont les segments longitudinaux extérieurs (7b) entourent par complémentarité de formes les surfaces de bridage (6c) de la partie supérieure (6).

12. Dispositif selon la revendication 11,
**caractérisé en ce que**
les ouvertures de sortie (3) se situent dans la partie inférieure (7).
